# EUROPEAN PATENT APPLICATION

(11) **EP 1 941 874 A1**
(43) Date of publication of application: **09.07.2008**
(21) Application number: 06797037.6
(22) Date of filing: 30.08.2006
(51) Int. Cl.: A61K 31/198, A61K 31/19, A61K 31/56, A61P 1/00, A61P 1/04, A61P 29/00

(54) **COLONIC DELIVERY-TYPE THERAPEUTIC AGENT FOR INFLAMMATORY BOWEL DISEASE**

(30) Priority: 30.08.2005 JP 2005249471
(71) Applicant: Ajinomoto Co., Inc., Tokyo 104-8315 (JP)
(72) Inventor: TAGAMI, Tomoyuki, Kanagawa 210-8681 (JP); TABATA, Tomoyuki, Tokyo 104-8315 (JP); UMEZAWA, Tsutomu, Tokyo 104-8315 (JP); SUZUKI, Yasuo, Chiba 262-0025 (JP)
(74) Representative: Nicholls, Kathryn Margaret
(86) International application number: PCT/JP2006/317062
(87) International publication number: WO 2007/026742

(57) **Abstract**

The present invention discloses a therapeutic agent for inflammatory bowel disease, which is in the from of delivering 1 to 5g of glutamine to the large intestine per one administration; and the therapeutic agent for inflammatory bowel disease with which an anti-inflammatory agent(s) is combined. This therapeutic agent for inflammatory bowel disease can safely and effectively treat inflammatory bowel disease including ulcerative colitis without increasing the frequency of bowel movements.

## Description

### Technical Field of the Invention

The present invention relates to therapeutic agents for inflammatory bowel disease which treat inflammatory bowel disease such as ulcerative colitis, colonic Crohn's disease and regional enteritis by delivering an active ingredient to the large intestine.

### Background of the Invention

Ulcerative colitis is an unexplained intractable chronic inflammatory disease, and definitive therapeutic method thereof has not yet been established. At present, as the drug therapy thereof, immunosuppressant agents such as cyclosporine are used in addition to steroids and 5-ASA anti-inflammatory agents. Among them, it is widely known that steroid therapy is useful, and steroid therapy is a main therapeutic method of ulcerative colitis. However, the method also has many problems such as unresponsiveness or dependency that happens in some of the patients. Further, it is pointed out that steroid therapy interrupts regeneration of mucosal tissues coupled with the strong anti-inflammatory action thereof (Non-patent Literatures 1 to 3). Under such circumstances, it has been desired to establish a therapeutic method for promoting regeneration of damaged mucosal tissues in a regional inflammatory part of the intestine and for promptly restoring the function of mucous membranes.

Meanwhile, L-glutamine which is an amino acid is an essential factor as a nutritional source of mucosal epithelial cells for proliferation, existence and functional maintenance of these cells. It is reported that L-glutamine has the mucosal-protective action in various digestive tract disorders (Non-patent Literatures 4 to 7). It is also illustrated that the dose of about 2g of L-glutamine a day is effective against epithelial injuries in the upper digestive tracts such as the stomach and the duodenum, and L-glutamine has been actually developed as a pharmaceutical product known as "Glumin^{®}" (Non-patent Literatures 8 and 9). However, when orally administering L-glutamine without dealing with the delivery technique, it is assumed that the efficiency of the delivery thereof to the lower digestive tracts without being absorbed or the efficiency of the delivery thereof to these tracts through blood is extremely low because of the prompt absorption and catabolization (metabolism) thereof in the upper digestive tracts and the catabolization (metabolism) thereof in the liver (Non-patent Literatures 10 to 12).

Though there are not many findings on usefulness of glutamine in the treatment of inflammatory bowel disease, it is reported that glutamine is effective in animal enteritis models (Non-patent Literatures 13 and 14). In these reports, rat models of TNBS are used and 0.25 to 0.4g/kg of L-glutamine is administered via the large intestinal route. Converting this amount to the amount when administered to humans, it becomes 15 to 24g. However, our study clarified that, when administering via the large intestinal route 10g of L-glutamine to humans, there is a problem that the frequency of bowel movements increases.

Meanwhile, it is also reported that GBF which comprises L-glutamine-rich proteins and hemicellulose as a food product for medical use, has an improving effect of the symptoms of DDS-induced colitis models (Non-patent Literature 15).
Non-patent Literature 1: Eubanks TR. et al., American Surgeon 1997; 63(3): 266-9
Non-patent Literature 2: Luo JC. et al., J Pharmacol Exp Ther. 2003; 307(2): 692-8
Non-patent Literature 3: Jung S. et al., Scand J Gastroenterol. 2001; 36(9): 963-70
Non-patent Literature 4: Savarese DMF. et al., Cancer Treatment Reviews 2003; 29: 501-13
Non-patent Literature 5: Deniele B. et al., Gut 2001; 48: 28-33
Non-patent Literature 6: Huffman FG. et al., HIV Clinical Trials 2003; 4(5): 324-29
Non-patent Literature 7: Rubio IT. et al., Annals of Surgery 1998; 227(5): 772-80
Non-patent Literature 8: Masayoshi Namiki et al., Chiryo (Therapeutics) 1974; 56(10): 177-86
Non-patent Literature 9: Yoshinori Nao et al., Rinsho-seijinbyo (Journal of Adult Diseases) 1979; 9(8): 187-94
Non-patent Literature 10: Brosnan JT., J Nutr. 2003; 133: 2068S-72S
Non-patent Literature 11: Ziegler TR. et al., J Parenter Enteral Nutr. 1990; 14(4 Suppl): 1375-146S
Non-patent Literature 12: Jebb SA. et al., Br J Cancer 1994; 70: 732-5
Non-patent Literature 13: Kaya E. et al., Dis Colon Rectum. 1999; 42(9): 1209-15
Non-patent Literature 14: Israeli E. et al., Dig Dis Sci. 2004; 49(10): 1705-1712
Non-patent Literature 15: Kanauchi O. et al., J Gastroenterol. 2003; 33: 179-88

### Disclosure of the Invention

The object of the present invention is to provide therapeutic agents for inflammatory bowel disease which can safely and effectively treat inflammatory bowel disease including ulcerative colitis without increasing the frequency of bowel movements.

The inventors thoroughly searched to solve the above problem and found that inflammatory bowel disease can be safely and effectively treated without having blood feces and increasing the frequency of bowel movements when delivering much less than 15g of L-glutamine to the large intestine of humans per one administration. They also found that the combined use of glutamine with an anti-inflammatory agent(s) significantly enhances the pathology improvement rate. The present invention has been completed based on these findings.

Namely, the present invention provides a therapeutic agent for inflammatory bowel disease which is in the form of delivering 1 to 5g of glutamine to the large intestine per one administration.

The present invention also provides the therapeutic agent for inflammatory bowel disease with which an anti-inflammatory agent(s) is combined.

### Brief Description of the Drawings

Figure 1 shows results of the drug efficacy test by enema administration of glutamine on DSS model in mice (referential example).
Figure 2 shows results of enema administration of 60mL of "Predonema®" Enema to which 10g of L-glutamine was added to patients with ulcerative colitis (comparative example 1).

### Best Mode for Carrying out the Invention

In the therapeutic agents for inflammatory bowel disease of the present invention, it is characterized in that the agents are in such forms that 1 to 5g and preferably 1.5 to 3g of glutamine reaches the large intestine per one administration. Glutamine may be D-form, L-form or DL-form, and L-glutamine is preferable. It may also be free forms or pharmaceutically acceptable salts thereof.

As such forms, enema agents are preferable. Since L-glutamine is unstable in a solution, the enema agents thereof can be prepared in the form wherein glutamine is mixed before use by using the same enema apparatus as that of "Predonema^{®}" Enema [comprising prednisolone sodium phosphate as the active ingredient] or that of "Pentasa^{®}" Enema [comprising mesalazine as the active ingredient], both of which are already marketed as enema agents for treating ulcerative colitis; or in the form of a kit preparation comprising glutamine powder and sterilized water for dissolution. More specifically, a pH adjuster(s) such as carboxy vinyl polymer, sodium hydrogenphosphate, ethyl parahydroxybenzoate, butyl parahydroxybenzoate, EDTA · Na, Na acetate and NaOH is dissolved in sterilized water; and then the above specified amount of L-glutamine can be added before use. Further, when formulating the enema agents of the present invention, an additive(s) used in common enema agents can be used as other combinational components. Such additives are not particularly limited, and it is possible to combine stabilizers, bases, suspending agents, emulsifying agents, thickening agents, dispersants, buffers, tonicity agents, pH adjusters, solubilizing agents, antiseptic agents, preservatives, antioxidants, solubilizing agents, dissolving agents, or the like.

The total amount of such enema agent can be determined considering usability, and 60 to 100mL thereof is preferable.

In this case, it is preferable in the present invention to use glutamine in combination with an anti-inflammatory agent(s).

As the anti-inflammatory agents, it is preferable to use steroids such as prednisolone (trade name: "Predonema^{®}", prednisolone tablets, or the like) and betamethasone (trade name: "Steronema^{®}" or the like); or 5-ASA agents such as mesalazine (trade name: "Pentasa^{®}" or the like) and salazosulfapyridine (trade name: "Salazopyrin^{®}" or the like). These anti-inflammatory agents can be administered via the oral or large intestinal route. The administered dose conforms to dosage and administration of each drug.

In the present invention, it is possible to further add the above specified amount of L-glutamine to a marketed enema agent for treating inflammatory bowel disease, for example, 60mL of "Predonema^{®}" Enema or 100mL of "Pentasa^{®}" Enema.

In the present invention, it is also possible to prepare the agents in the form of oral agent that releases glutamine in the large intestine. For example, it is possible to prepare the agent wherein a core agent comprising glutamine is coated with hydrophobic organic compound - enteric polymer mixed film as mentioned in JP 2000-103732 A, such as stearic acid - EudragitL100 mixed film. More specifically, the agent can be produced in accordance with Example 2 of JP 2000-103732 A. Further, it is also possible to produce the agent by coating a core agent comprising glutamine with films mentioned in JP 2917799 B and JP 3185206 B. The descriptions of these patents and the patent application are included in that of the specification of the present invention. Meanwhile, in the case of an oral agent, it preferably comprises a necessary amount of glutamine so that 1 to 5g of glutamine reaches the large intestine per one administration. For example, 1 to 30g of glutamine is preferably administered as the converted dose of glutamine.

In the above oral agent, it is possible to add to the core agent, in addition to glutamine, excipients such as lactose, corn starch, sucrose, glucose, sorbit and crystalline cellulose; binders such as polyvinyl alcohol, polyvinyl ether, ethylcellulose, methylcellulose, gum arabic, tragacanth, gelatin, shellac, hydroxypropylcellulose, hydroxypropylstarch and polyvinyl pyrrolidone; and disintegrating agents such as starch, agar, gelatin powder, crystalline cellulose, calcium carbonate, sodium hydrogen carbonate, calcium citrate, dextran and pectin.

In order to making the agent easier to swallow, the outside of the above coated layer may be further arbitrarily coated with sugar, gelatin, or the like, which comprises or does not comprise a flavoring and freshening substance such as cocoa powder, menthol, aromatic acids, peppermint oil, borneol, cinnamon powder and the like. When formulating the oral solid preparations of the present invention, an additive(s) used in common oral preparations can be used as other combinational components. Such additives are not particularly limited, and it is possible to combine excipients such as sugars, starches and crystalline cellulose; binders; lubricants such as magnesium stearate and tare; pH adjusters; coloring agents; and flavoring agents such as sugars and menthols.

The therapeutic agents for inflammatory bowel disease of the present invention can be preferably used for treating inflammatory bowel disease such as ulcerative colitis, colonic Crohn's disease and regional enteritis. It is particularly preferable to use the agents for treating ulcerative colitis.

Besides, the therapeutic agents for inflammatory bowel disease of the present invention are useful for patients with the above diseases. Especially, they are useful for the patients with ulcerative colitis whose pathologies do not improve by steroid therapy, or whose symptoms do not completely heal by steroid therapy.

Next, Examples will further illustrate the present invention.

### Examples

### Referential Example: Results of a drug efficacy test by enema administration of glutamine on DSS model in mice

5%DSS water (dissolving dextran sulfate sodium in tap water) was drank ad lib by a male C57BL/6 Cr S1c mouse of 8 weeks old, and it developed ulcerative colitis. From one day before starting drinking of DSS water, L-glutamine was administered via the large intestinal route to the mouse once a day. An enema agent (solution) having the composition of 0.5 % (w/v) carboxymethylcellulose - sodium was used as the enema agent.

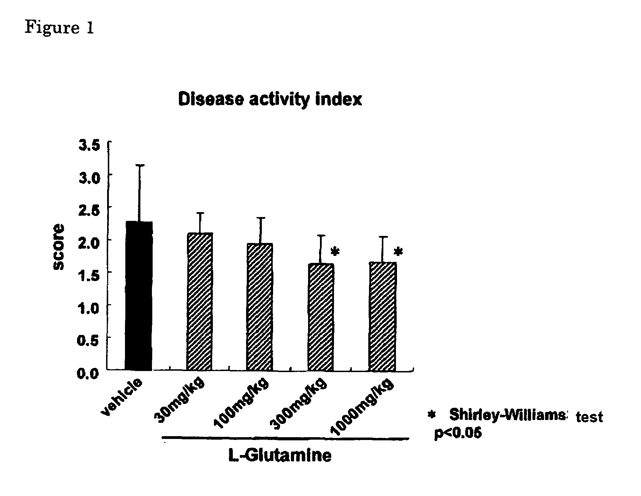

As the disease activity index, each average value of body weight scores, blood feces scores and diarrhea scores was calculated referring to the report of HS. Cooper et al. (Laboratory Investigation 1993; 69(2): 238). Figure 1 shows results of the sixth day after the start of drinking DSS water.

As obvious from the results of Figure 1, there was no significant difference between L-glutamine (30 and 100 mg/kg)-treated groups and vehicle-treated group. Namely, it indicates that 1.8g (corresponding to 30mg/kg) or 6g (corresponding to 100mg/kg) which is the converted amount when administered to humans would not have a therapeutic effect against ulcerative colitis.

### Comparative Example 1

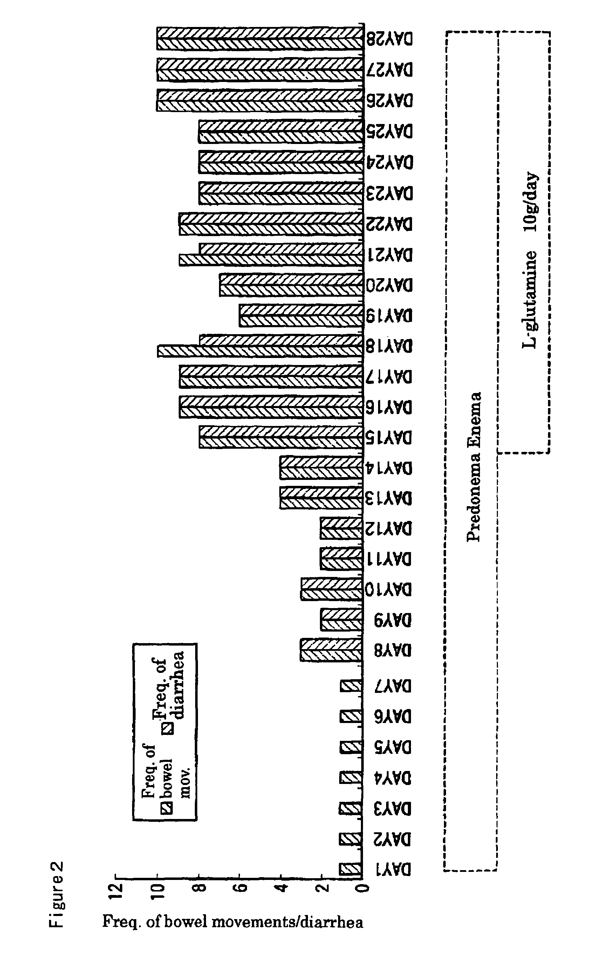

10g of L-glutamine was added to 60mL of "Predonema®" Enema, and the mixture was administered via the large intestinal route to patients with ulcerative colitis. The frequencies of bowel movements and diarrhea were measured. Figure 2 shows results thereof. Meanwhile, the patients were treated with "Predonema^{®}" from Day1 to Dayl4, and 60mL of "Predonema^{®}" Enema prepared by adding 10g of L-glutamine thereto was administered via the large intestinal route to the patients from Day 15 to Day 28.

Though an improving effect of blood feces was recognized from the observation of feces, the frequency of bowel movements increased in accordance with the administration of glutamine as shown in Figure 2.

### Example 1

2g of L-glutamine was added to 60mL of "Predonema^{®}" Enema, and the mixture was administered via the large intestinal route to patients with ulcerative colitis whose pathologies do not improve by a therapy with "Predonema^{®}", or whose symptoms do not completely heal by a therapy with "Predonema^{®}" (Case Nos. 1, 2, 3, 4, 6, 7, and 8).

Further, 60mL of an enema agent prepared by adding 2g of L-glutamine to sterilized water was administered via the large intestinal route to the patients with ulcerative colitis having the same symptoms as mentioned above (Case Nos. 5 and 9 to 14).

In addition, the patients with ulcerative colitis of Comparative Example and Example take a 5-ASA agent(s) ("Pentasa^{®}" or "Salazopyrin^{®}") as an oral agent conforming to dosage and administration.

The frequencies of bowel movements and diarrhea, and blood feces scores were measured of these patients. Table 1 shows results thereof.

**Table 1**

| Case No. | Admin. Period | Freq. of bowel movement (freq. of diarrhea)^{*1} | | Blood feces score^{*2} | | Prescription (all 5-ASA agent) |
|---|---|---|---|---|---|---|
| | | Bef. admin. | Aft. admin. | Bef. admin. | Aft. admin. | |
| No. 1 | 14 | 5.4 (ND^{*3}) | 6.1 (5.7) | 3.0 | 1.3 | "Predonema" + Gln |
| No.2 | 23 | 2.3 (0.9) | 3.1(1.1) | 1.0 | 0.0 | "Predonema" + Gln |
| No.3 | 26 | 6.0 (0.0) | 4.1(1.0) | 0.9 | 0.6 | "Predonema" + Gln |
| No.4 | 39 | 3.7 (0.3) | 3.9 (0.1) | 0.3 | 0.0 | "Predonema" + Gln |
| No.5 | 26 | 4.5 (2.0) | 5.0 (0.1) | 2.2 | 1.3 | Gln |
| No.6 | 14 | 6.5 (1.6) | 6.6 (2.3) | 1.0 | 0.9 | "Predonema" + Gln |
| No.7 | 14 | 3.2 (2.2) | 3.1 (0.0) | 1.4 | 0.8 | "Predonema" + Gln |
| No.8 | 14 | 2.7 (0.1) | 3.4 (0.0) | 1.8 | 1.0 | "Predonema" + Gln |
| No.9 | 21 | 5.4 (0.9) | 5.3 (0.4) | 1.3 | 1.0 | Gln |
| No.10 | 21 | 2.5 (0.0) | 2.3 (0.0) | 0.8 | 0.8 | Gln |
| No.11 | 21 | 4.2 (1.5) | 3.9 (2.6) | 1.3 | 0.9 | Gln |
| No.12 | 21 | 6.7 (0.0) | 4.1 (0.0) | 1.0 | 1.0 | Gln |
| No.13 | Stopped for aggravation of symptoms after starting administration | | | | | Gln |
| No.14 | Stopped for aggravation of symptoms after starting administration | | | | | Gln |

| | | | | | | |
|---|---|---|---|---|---|---|
| Remarks) *1: The frequency of bowel movements is an average value per a day of each period, and the number in 0 indicates the frequency of diarrhea. *2: Blood feces score indicates an average value per a day of each administration period as setting no blood feces: 0; less blood feces: 1; and a lot of blood feces: 3. *3: no data | | | | | | |

From the results of Table 1, improvement in blood feces was recognized in all cases. Administration of L-glutamine not only maintained the frequency of bowel movements but also was able to improve diarrhea as shown in Case Nos. 4 and 5.

Further, as shown in Table 1, when administering glutamine without "Predonema", the blood feces improved in 3 out of 7 cases (improvement rate: 43%). On the other hand, when administering glutamine in combination with "Predonema" which is an anti-inflammatory agent, the blood feces improved in 7 out of 7 cases (improvement rate: 100%). Thus, it is obvious that the combined use of glutamine with "Predonema" which is the anti-inflammatory agent significantly increases the improvement rate of pathology.

## Claims

1. A therapeutic agent for inflammatory bowel disease, which is in the form of delivering 1 to 5g of glutamine to the large intestine per one administration.

2. The therapeutic agent for inflammatory bowel disease according to claim 1, which is in the form of delivering 1.5 to 3g of glutamine to the large intestine.

3. The therapeutic agent for inflammatory bowel disease according to claim 1 or 2, which is in the form of an enema agent.

4. The therapeutic agent for inflammatory bowel disease according to claim 1 or 2, which is in the form of an oral agent that releases glutamine in the large intestine.

5. The therapeutic agent for inflammatory bowel disease according to any one of claims 1 to 4, wherein the inflammatory bowel disease is ulcerative colitis, colonic Crohn's disease or regional enteritis.

6. The therapeutic agent for inflammatory bowel disease according to any one of claims 1 to 5 with which an anti-inflammatory agent(s) is combined.

7. The therapeutic agent for inflammatory bowel disease according to claim 6, wherein the anti-inflammatory agent is a steroid and/or a 5-ASA agent.
